# EUROPEAN PATENT APPLICATION

(11) **EP 4 549 425 A1**
(43) Date of publication of application: **07.05.2025**
(21) Application number: 23830562.7
(22) Date of filing: 22.06.2023
(51) Int. Cl.: C07C 50/04, A61K 31/122, A61P 33/02

(54) **QUINONE-DERIVED COMPOUNDS AND USE THEREOF AGAINST LEISHMANIA SPP**

(30) Priority: 28.06.2022 ES 202230581
(71) Applicant: Universidad de la Laguna, 38508 La Laguna (ES)
(72) Inventor: ANA RAQUEL DÍAZ MARRERO, Ana, 38200 San Cristóbal de La Laguna (ES); JOSÉ JAVIER FERNÁNDEZ CASTRO, Javier, 38200 San Cristóbal de La Laguna (ES); SARA GARCÍA DAVIS, Sara, 38200 San Cristóbal de La Laguna (ES); CARLOS JAVIER ESTRELLA BETHENCOURT, Carlos, 38200 San Cristóbal de La Laguna (ES); ATTENERI LÓPEZ ARENCIBIA, Atteneri, 38200 San Cristóbal de La Laguna (ES); JOSÉ PIÑERO BARROSO, José, 38200 San Cristóbal de La Laguna (ES); JACOB LORENZO MORALES, Jacob, 38200 San Cristóbal de La Laguna (ES)
(74) Representative: Isern Patentes y Marcas S.L.
(86) International application number: PCT/ES2023/070407
(87) International publication number: WO 2024/003428

(57) **Abstract**

The present invention relates to quinone-derived compounds with leishmanicidal activity and to the use of same in the pharmaceutical industry, specifically in the field of parasitic diseases. The invention relates specifically to a compound of formula (I), the isomeric forms thereof and the salts of same, wherein R1 and R3 are hydrogen and R2 is selected from the list consisting of cyclopentyl, cycloheptyl, 3-methyl-pentyl and adamantane, or wherein R1 and R2 are hydrogen and R3 is selected from the list consisting of cyclopentyl, cyclohexyl and cycloheptyl.

## Description

### TECHNICAL FIELD

The present invention relates to quinone-derived compounds and the use of same in the pharmaceutical sector, specifically in the field of parasitic diseases.

### BACKGROUND OF THE INVENTION

Protozoan parasitic diseases represent a major global public health problem, costing developing economies billions of dollars every year (WHO, 2017). Leishmaniasis is caused by a protozoan parasite comprising 20 species of *Leishmania* and is classified into three different clinical forms: visceral, mucocutaneous and cutaneous. The latter is the most common form, with 600,000 to 1 million new cases being estimated annually worldwide (WHO), 2021).

Leishmaniasis is curable if diagnosed early and if appropriate medication is administered. The treatment of first choice is based on pentavalent antimony salts, such as methylglucamine antimoniate and sodium stibogluconate. Other medicinal products such as miltefosine are used as second-line treatment (Copeland and Aronson, 2015). However, miltefosine has a high toxicity and resistance, as well as being a long and expensive treatment; therefore, the search for safer, more effective and more economical treatments is required (Oryan, 2015).

Cutaneous leishmaniasis, the lesions of which are characterised by nodules that can destroy the epidermis and thus result in ulcers (Peralta *et al.* 2021), can be successfully treated with variable results between 55 and 98%, depending on the geographical area and the species causing the disease (Corpas-López *et al.* 2016). It is endemic in many developing countries, mainly due to the limited number of medicinal products available to patients. Furthermore, most treatments require parenteral administration, resulting in low patient compliance, high costs and consequently worse results (Azim *et al.* 2021). Although topical paromomycin, amphotericin B and miltefosine preparations have been developed and evaluated, sufficiently effective formulations have not been obtained (Corpas-López *et al.* 2016).

In recent years, much emphasis has been placed on the development of topical alternatives that are easy to apply and do not generate systemic toxicity, observing certain preference for some types of molecules, such as those having a quinone nucleus, which often acts as a DNA intercalating agent, a biomolecule alkylating agent, and/or a reactive oxygen species generator (Ibacache *et al.,* 2018).

Some quinone-type compounds obtained by synthesis have also been described (Gunatilaka, A.A.; Berger, J.M.; Evans, R.; Miller, J.S.; Wisse, J.H.; Neddermann, K.M.; Bursuker, I.; Kingston, D.G. Isolation, Synthesis, and Structure-Activity Relationships of Bioactive Benzoquinones from Miconia lepidota from the Suriname Rainforest. J. Nat. Prod. 2001, 64,1, 2-5) or by derivatisation of natural products comprising this fragment, for example, from perezone, a natural quinone used as the basis for synthesising a series of cytotoxic derivatives, have also been described. However, although IC50 values between 3.3 and 24.8 µM have been described (Concepción-Lozada, M.; Soria-Arteche, O.; Ramírez-Apan, M.T.; Nieto-Camacho, A.; Enriquez, R.G.; Izquierdo, T.; Jiménez-Corona, A. Synthesis, cytotoxic and antioxidant evaluations of amino derivatives from perezone. Bioorg. Med. Chem. 2012, 20, 17, 5077-5084.), more optimal IC50 values for leishmanicidal treatment would be less than 1 µg/ml (Nwaka, S.; Hudson, A. Innovative lead discovery strategies for tropical diseases. Nat. Rev. Drug Discov. 2006, 5, 11, 941-955; Nwaka, S.; Ramírez, B.; Brun, R.; Maes, L.; Douglas, F.; Ridley, R. Advancing drug innovation for neglected diseases-criteria for lead progression. PLoS Negl. Trop. Dis. 2009, 3, 8, e440).

Accordingly, there is a need to provide effective and low-toxicity treatments for leishmaniasis that can overcome the aforementioned drawbacks.

### BRIEF DESCRIPTION OF THE INVENTION

The present invention, therefore, solves the problems addressed. In this way, the first aspect of the invention relates to a compound of formula (I), the isomeric forms thereof and the salts of same, 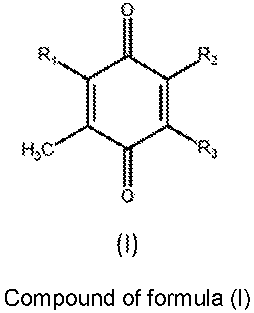
wherein R1 and R3 are hydrogen and R2 is selected from the list consisting of cyclopentyl, cycloheptyl and 3-methyl-pentyl;
or wherein R1 and R2 are hydrogen and R3 is selected from the list consisting of cyclopentyl, cyclohexyl and cycloheptyl.

The compounds of formula (I) according to the first aspect of the invention exhibit activity against leishmaniasis, showing a good efficacy and a low toxicity. The compounds of formula (I) according to the first aspect have IC₅₀ values < 1 µg/ml, which is a significant improvement over the reference drug miltefosine, with an IC₅₀ of 2.64 µg/ml.

The second aspect of the invention relates to compounds of formula (I), their isometric forms and salts thereof, for use as a medicinal product, 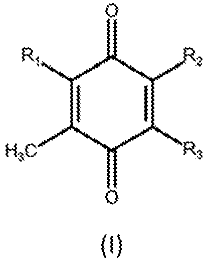
wherein R1 and R3 are hydrogen and R2 is selected from the list consisting of cyclopentyl, cyclohexyl, cycloheptyl, 3-methyl-pentyl, isopentanyl or adamantane;
or wherein R1 and R2 are hydrogen and R3 is selected from the list consisting of cyclopentyl, cyclohexyl and cycloheptyl.

The compounds of formula (I) according to the second aspect of the invention exhibit activity against leishmaniasis, showing a good efficacy and a low toxicity. For example, the compounds of formula (I) according to the second aspect of the invention exhibit IC₅₀ values < 1 µg/ml, which is an improvement over the reference drug miltefosine, which has an IC₅₀ of 2.64 µg/ml.

Taking into account that one of the main limitations in conducting pre-clinical and clinical studies of potential therapeutic agents is the difficult access to the natural source, the third aspect of the invention relates to the synthesis of the compounds of the first and second aspects of the invention.

The synthetic method can be approached through two methodologies comprising at least two reaction steps and can be carried out from readily available and low-cost commercial compounds, as described in diagram 1. 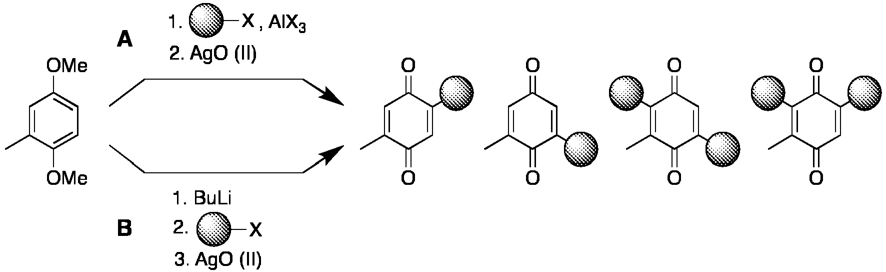
- Method A.
   Step 1: Friedel and Craft alkylation of 2,5-dimethoxytoluene using substituted halides in the presence of AlCl₃.
   Step 2: Deprotection reaction by means of oxidation with AgO (II).
- Method B.
   Step 1: Coupling reaction of the fragment of interest by reacting alkyl halides with the carbanion obtained by treating 2,5-dimethoxytoluene in the presence of BuLi.
   Step 2: Deprotection reaction by means of oxidation with AgO (II).

The compounds according to the first aspect obtained by means of these two synthetic methodologies, the third and fourth aspects of the invention, have made it possible to obtain the compounds of formula (I) of the first and second aspects in order to subsequently be able to evaluate the anti-leishmanial activity. These compounds have been obtained with a good yield and with a purity of more than 95%, preferably more than 99%, which makes them suitable for medical use.

The fifth aspect of the invention relates to a pharmaceutical composition comprising the compound of formula (I) according to the first or second inventive aspect or to the compounds obtained from the third aspect of the invention, in addition to a pharmaceutically acceptable excipient.

### BRIEF DESCRIPTION OF THE DRAWINGS

The following graphical description is given by way of non-limiting example in order to detail the features and advantages of the invention.

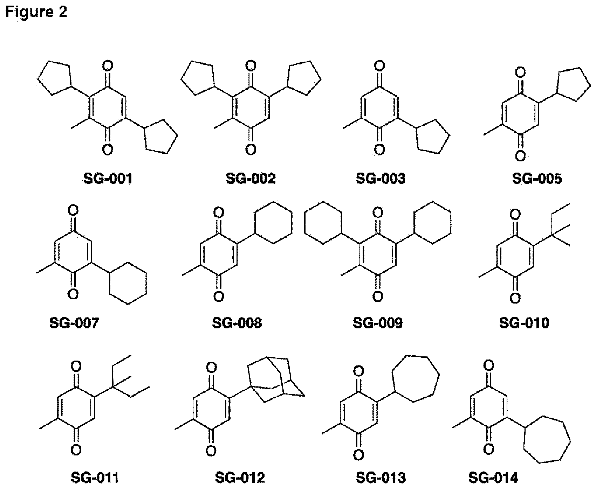

**Figure 2****.** Chemical structure of synthetic quinones. The names of the compounds are specified in the following table:

| | |
|---|---|
| 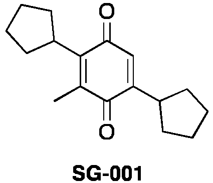 | 2,5-dicyclopentyl-3-methylcyclohexa-2,5-diene-1,4-dione |
| 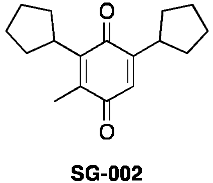 | 3,5-dicyclopentyl-2-methylcyclohexa-2,5-diene-1,4-dione |
| 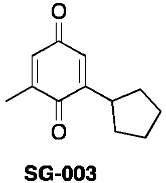 | 2-cyclopentyl-6-methylcyclohexa-2,5-diene-1,4-dione |
| 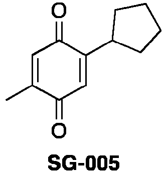 | 2-cyclopentyl-5-methylcyclohexa-2,5-diene-1,4-dione |
| 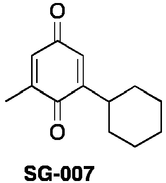 | 2-cyclohexyl-6-methylcyclohexa-2,5-diene-1,4-dione |
| 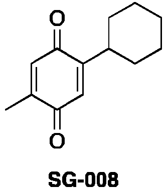 | 2-cyclohexyl-5-methylcyclohexa-2,5-diene-1,4-dione |
| 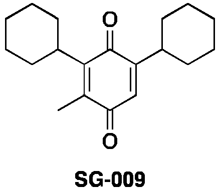 | 3,5-dicyclohexyl-2-methylcyclohexa-2,5-diene-1,4-dione |
| 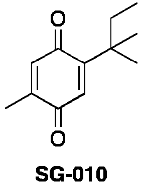 | 2-methyl-5-(tert-pentyl)cyclohexa-2,5-diene-1,4-dione |
| 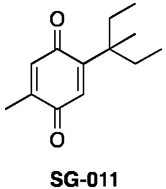 | 2-methyl-5-(3-methylpentan-3-yl)cyclohexa-2,5-diene-1,4-dione |
| 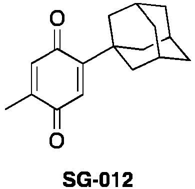 | 2-((3r,5r,7r)-adamantane-1-yl)-5-methylcyclohexa-2,5-diene-1,4-dione |
| 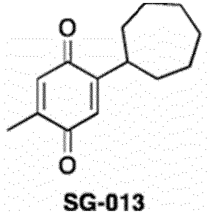 | 2-cycloheptyl-5-methylcyclohexa-2,5-diene-1,4-dione |
| 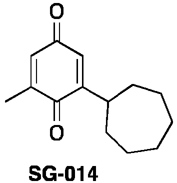 | 2-cycloheptyl-6-methylcyclohexa-2,5-diene-1,4-dione |

### DETAILED DESCRIPTION OF THE INVENTION

The first aspect of the invention relates to the compounds of formula (I), the isomeric forms thereof and the salts of same, 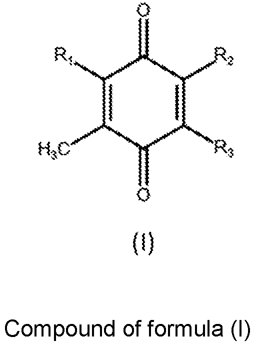
wherein R1 and R3 are hydrogen and R2 is selected from the list consisting of cyclopentyl, cycloheptyl and 3-methyl-pentyl;
or wherein R1 and R2 are hydrogen and R3 is selected from the list consisting of cyclopentyl, cyclohexyl and cycloheptyl.

The compounds of formula (I) according to the first aspect of the invention exhibit activity against leishmaniasis, showing a good efficacy and a low toxicity. The compounds of formula (I) according to the first aspect have IC₅₀ values < 1 µg/ml, which is a significant improvement over the reference drug miltefosine, with an IC₅₀ of 2.64 µg/ml.

In a preferred embodiment of the first aspect, the compounds of formula I are selected from the list of structures consisting of: 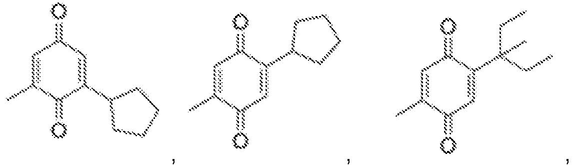 or

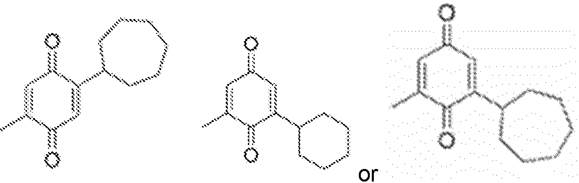

The second aspect of the invention relates to compounds of formula (I), their isometric forms and salts thereof, for use as a medicinal product, 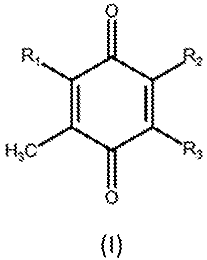
wherein R1 and R3 are hydrogen and R2 is selected from the list consisting of cyclopentyl, cyclohexyl, cycloheptyl, 3-methyl-pentyl, isopentanyl or adamantane;
or wherein R1 and R2 are hydrogen and R3 is selected from the list consisting of cyclopentyl, cyclohexyl and cycloheptyl.

The compounds of formula (I) according to the second aspect of the invention exhibit activity against leishmaniasis, showing a good efficacy and a low toxicity. For example, the compounds of formula (I) according to the second aspect of the invention exhibit IC₅₀ values < 1 µg/ml, which is an improvement over the reference drug miltefosine, which has an IC₅₀ of 2.64 µg/ml.

In a preferred embodiment of the second aspect of the invention, the compound of formula I is selected from the list of structures consisting of: 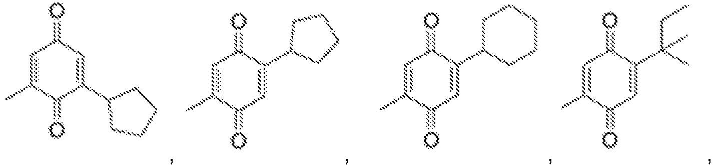 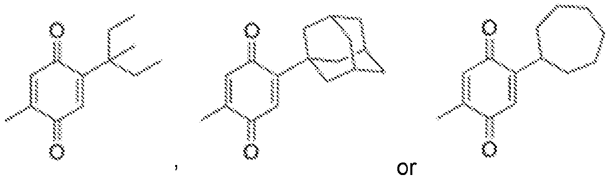 or

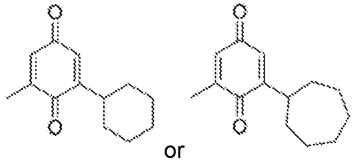

A preferred embodiment of the second aspect of the invention relates to the compounds of formula (I) for use as a human or veterinary medicinal product.

A preferred embodiment of the second aspect relates to compounds of formula (I) for use in the treatment of leishmaniasis.

In a more preferred embodiment of the second aspect of the invention, leishmaniasis is caused by *Leishmania spp.*

The third aspect of the invention relates to a method of obtaining the compound of formula (I) according to the first aspect or the compound of formula (I) according to the second aspect, wherein the method comprises at least the following steps:
i) providing a mixture of 2,5-dimethoxytoluene in the presence of aluminium trichloride and alkyl chloride in a nitrogenous solvent, preferably nitromethane;
ii) stirring the mixture from the previous step for at least 30 minutes at a temperature range of -10°C to 10°C, preferably 0°C, under anhydrous conditions and in an inert atmosphere;
iii) adding water to the product of the previous reaction to stop the reaction followed by extraction of the products obtained in the previous step with an organic solvent;
iv) adding silver oxide and nitric acid sequentially to the products obtained in the previous step;
v) optionally adding water to the product of the previous reaction followed by organic solvent extraction;
vi) optionally, performing a purification step.

Other solvents were tested in step i), such as ether, THF, hexane or pentane, however, said solvents gave poor results at least in relation to the reaction yield.

In a preferred embodiment of the invention according to the third aspect of the invention, the organic solvent of step iii) is selected from a list consisting of hexane, pentane, ethyl acetate or dichloromethane.

In a preferred embodiment of the invention according to the third aspect of the invention, the organic solvent of step v) is selected from the list consisting of dichloromethane, hexane, diethyl ether, acetone, methanol, ethanol, isopropanol tetrahydrofuran and diethyl amine.

The fourth aspect of the invention relates to a method of obtaining the compound of formula (I) according to the first aspect or the compound of formula (I) according to the second aspect, wherein the method comprises at least the following steps:
i) providing a mixture of 2,5-dimethoxytoluene organic solvent at a temperature in the range of -5°C to 15°C, preferably 0-10°C;
ii) adding to the previous mixture a solution of n-butyllithium in organic solvent at a temperature in a range of -5°C to 10°C for at least 10 minutes; preferably 0°C for 10 minutes;
iii) increasing the temperature of the previous mixture to a range of 20°C to 30°C;
iv) cooling the product from the previous step to a temperature in the range of -10°C to 5°C, followed by the dropwise addition of alkyl chloride;
v) adding ammonium chloride to the product from the previous step, followed by organic solvent extraction;
vi) adding silver oxide and nitric acid sequentially to the products obtained in the previous step;
vii) optionally adding water to the product of the previous reaction followed by organic solvent extraction;
viii) optionally performing a purification step.

In a preferred embodiment of the invention according to the fourth aspect of the invention, the organic solvent of step i) is selected from hexane and acetone.

In another preferred embodiment of the invention according to the fourth aspect of the invention, the alkyl of step iv) is a linear or branched C1-C10 alkyl.

In another preferred embodiment of the invention according to the fourth aspect of the invention, the organic solvent of steps i) and vii) is selected from the list consisting of dichloromethane, hexane, diethyl ether, acetone, methanol, ethanol, isopropanol tetrahydrofuran and diethyl amine.

The fifth aspect of the invention relates to a pharmaceutical composition comprising the compound of formula (I) according to the first or second inventive aspect or to the compounds obtained from the third and fourth aspects of the invention, in addition to a pharmaceutically acceptable excipient.

In a preferred embodiment of the fifth aspect, the pharmaceutical composition is for topical or oral application.

In another preferred embodiment of the fifth aspect, the pharmaceutical composition of the fifth aspect is for topical application and is in liquid form, in cream form or in gel form.

In a preferred embodiment of the third aspect, the pharmaceutical composition is for oral application in tablet, capsule, granule, pill or freeze-dried form.

In another more preferred embodiment of the fifth aspect, the pharmaceutical composition is for topical application, preferably in gel form or in cream form.

In a preferred embodiment of the fifth aspect of the invention, the compound of formula (I) is present in the pharmaceutical composition in a range of 0.1-5% w/w (by weight).

In another preferred embodiment of the fifth aspect of the invention, the composition comprises:
0.1-5% by weight of the compound of formula (I),
35-45% by weight of polyacrylamide,
15-25% by weight of C13-14 isoparaffin,
3-8% surfactant, the surfactant preferably being laureth-7.

### EXAMPLES

### Example 1. Obtaining compounds of FORMULA I (SG-013, wherein R1 and R3 are H and R2 is cycloheptane) with leishmanicidal activity

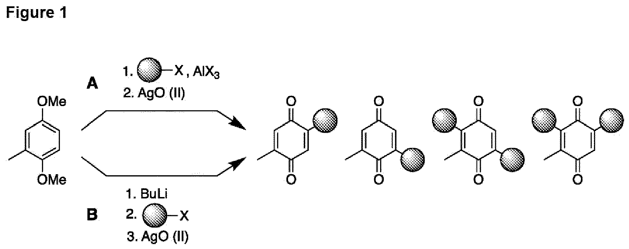

The first step in obtaining the compounds of formula (I) SG-013, wherein R1 and R3 are H and R2 is cycloheptane, consists of the formation of 2-methyl substituted aromatic substances (method A, Figure 1). To do so, a solution of 2,5-dimethoxytoluene (3.285 mmol) was reacted, in the presence of aluminium trichloride (6.57 mmol) and chlorocycloheptane (6.57 mmol), dissolved in nitromethane, anhydrous conditions and an inert atmosphere. The reaction is kept under stirring at 0°C for 1 hour. After adding water, the reaction products were extracted with ether (Williamson 1999).

Next, liquid-liquid extraction was performed with ethyl ether and it was dried with MgSO₄. The solvent was evaporated under reduced pressure and the product was fractionated by means of silica gel column chromatography (150 x 30 mm Ø) using *n*-hex:AcOEt mixtures (99:1 - 97:3) as an eluent. The fractions obtained were concentrated, and the fraction that showed signs of 2,5-dimethoxytoluene ring substitution was identified according to ¹H NMR spectra.

The next reaction step consists of deprotection of the methoxyl groups and formation of the quinones by adding silver(II) oxide (4.84 mmol) dissolved in 7 N nitric acid (0.2 ml) to a solution of 300 mg of the intermediate products in 4 ml dioxane. The reaction was kept under stirring at 0°C for 1 hour, stopped with water and extracted with dichloromethane (Sanchez *et al.* 1985).

The product was fractionated by means of silica column chromatography (150 x 15 mm Ø) using *n*-hex:AcOEt under isocratic conditions (99:1) as an eluent. The fraction showing signs of benzoquinones was selected according to the ¹H NMR spectra of the obtained fractions. The fraction of interest was purified by means of HPLC using a silica column (250 x 10 mm Ø) with gradient *n*-Hex:AcOEt (99:1 - 0:100) as an eluent.

The yield of the reaction was 49% and the purity of SG-013 is 95%. The compound was characterised by nuclear magnetic resonance and confirmed by mass spectrometry.

The compound SG-013 was obtained as a translucent crystal; HRESIMS *m*/*z* 217.1234 [M-H]-(calc. C₁₄H₁₈O₂, 217.1234) ¹H NMR (600 MHz, CDCl₃) δ 1.40 (1H, m, H-9), 1.40 (1H, m, H-10), 1.55 (1H, m, H-10), 1.65 (1H, m, H-11), 1.75 (1H, m, H-9), 1.75 (1H, m, H-10), 2.02 (3H, d, J=0.92, H-7), 2.84 (1H, tt, J=10.6, 3.1, H-8), 6.49 (1H, s, H-3), 6.57 (1H, d, J=0.92, H-6); ¹³C NMR (125 MHz, CDCl₃) δ 15.53 (C-7), 27.05 (C-10), 27.92 (C-11), 34.25 (C-9), 37.79 (C-8), 130.68 (C-3), 133.86 (C-6), 145.26 (C-5), 155.35 (C-2), 187.65 (C-1), 188.93 (C-4).

The synthesised compounds are detailed in Figure 2.

### Example 2. Obtaining compounds of FORMULA I with leishmanicidal activity

In another embodiment of the invention, the compounds of formula (I) have been prepared by means of the following method B as described in Figure 1:
Obtaining the compounds of formula (I) SG-013, wherein R1 and R3 are H and R2 is cycloheptane, started with a solution of 2,5-dimethoxytoluene (50.0 mg, 0.3 mmol) in 10 ml of ether at a temperature of 0°C, and a solution of 0.5 ml of 1.7 M n-butyllithium (0.5 mmol) in hexane was added. After 10 minutes at 0°C, the reaction was kept under stirring for 24 hours at room temperature. Subsequently, the mixture was cooled to 0°C again, and chlorocycloheptane was added dropwise. After 30 minutes of stirring, the reaction was stopped with a saturated ammonium chloride solution, diluted with water and extracted with ethyl acetate (Sanchez *et al.* 1985).

Next, liquid-liquid extraction was performed with ethyl ether and it was dried with MgSO₄. The solvent was evaporated under reduced pressure and the product was fractionated by means of silica gel column chromatography using *n*-hex:AcOEt (99:1 - 97:3) as an eluent. The fractions obtained were concentrated, and the fraction that showed signs of 2,5-dimethoxytoluene ring substitution was identified according to ¹H NMR spectra.

As in Example 1, the last reaction step consists of deprotection of the methoxyl groups and formation of quinones by adding silver oxide dissolved in nitric acid. The resulting products (compounds of formula I) were purified by means of silica column chromatography, followed by purification via HPLC (liquid chromatography) using *n*-hex:AcOEt (99:1 - 0:100) as an eluent. Identification was performed by means of nuclear magnetic resonance spectroscopic techniques and confirmed by mass spectrometry.

The compound SG-013 was obtained as a translucent crystal; HRESIMS m/z 217.1234 [M-H]-(calc. C₁₄H₁₈O₂, 217.1234) ¹H NMR (600 MHz, CDCl₃) δ 1.40 (1H, m, H-9), 1.40 (1H, m, H-10), 1.55 (1H, m, H-10), 1.65 (1H, m, H-11), 1.75 (1H, m, H-9), 1.75 (1H, m, H-10), 2.02 (3H, d, J=0.92, H-7), 2.84 (1H, tt, J=10.6, 3.1, H-8), 6.49 (1H, s, H-3), 6.57 (1H, d, J=0.92, H-6); ¹³C NMR (125 MHz, CDCl₃) δ 15.53 (C-7), 27.05 (C-10), 27.92 (C-11), 34.25 (C-9), 37.79 (C-8), 130.68 (C-3), 133.86 (C-6), 145.26 (C-5), 155.35 (C-2), 187.65 (C-1), 188.93 (C-4).

Example 1. Obtaining compounds of FORMULA I (SG-010, wherein R1 and R3 are H and R2 is 2-methylbutane) with leishmanicidal activity.

The first step in obtaining the compounds of formula (I) SG-010, wherein R1 and R3 are H and R2 is 2-methylbutane, consists of the formation of 2-methyl substituted aromatic substances (method A, Figure 1). To do so, a solution of 2,5-dimethoxytoluene (3.28 mmol) was reacted, in the presence of aluminium trichloride (6.57 mmol) and 2-chloro-2-methylbutane (6.57 mmol), dissolved in nitromethane, under anhydrous conditions and in an inert atmosphere. The reaction was kept under stirring at 0°C for 1 hour. After adding water, the reaction products were extracted with ether (Williamson 1999).

Next, liquid-liquid extraction was performed with ethyl ether and it was dried with MgSO₄. The solvent was evaporated under reduced pressure and the product was fractionated by means of silica gel column chromatography (120 x 30 mm Ø) using *n*-hex:AcOEt (99:1 - 97:3) as an eluent. The fractions obtained were concentrated, and the fraction that showed signs of 2,5-dimethoxytoluene ring substitution was identified according to ¹H NMR spectra.

The next reaction step consists of deprotection of the methoxyl groups and formation of the quinones by adding silver(II) oxide (5.34 mmol) dissolved in 7 N nitric acid (2 ml) to a solution of 294.0 mg of the intermediate products in 4 ml dioxane. The reaction was kept under stirring at 0°C for 1 hour, stopped with water and extracted with dichloromethane (Sanchez *et al.* 1985).

The product was fractionated by means of silica column chromatography (120 x 30 mm Ø) using *n*-hex:AcOEt under isocratic conditions (99:1) as an eluent. The fraction showing signs of benzoquinones was selected according to the proton spectra of the obtained fractions. The fraction of interest was purified by means of HPLC using a silica column (250 x 10 mm Ø) with gradient *n*-Hex:AcOEt (99:1 - 0:100) as an eluent.

The yield of the reaction was 37% and the purity of SG-010 is 95%. The compound was characterised by nuclear magnetic resonance and confirmed by mass spectrometry.

The compound SG-010 was obtained as a translucent crystal; HRESIMS m/z 191.1072 [M-H]-(calc. C₁₂H₁₅O₂, 191.1072) ¹H NMR (600 MHz, CDCl₃) δ 0.87 (3H, m, H-11), 1.20 (3H, s, H-8), 1.20 (3H, s, H-12), 1.70 (2H, dd, J = 5.0, 3.6 Hz, H-10), 2.01 (3H, d, J = 1.6 Hz, H-7), 6.52 (1H, s, H-6); ¹³C NMR δ 15.2 (C-7), 27.8 (C-8), 27.8 (C-12), 30.2 (C-11), 35.7 (C-10), 38.5 (C-9), 133.3 (C-6), 135.5 (C-3), 144.3 (C-2), 155.2 (C-5), 187.9 (C-4), 188.8 (C-1).

The synthesised compounds are detailed in Figure 2.

The compound SG-010, wherein R1 and R3 are H and R2 is cycloheptane and shows leishmanicidal activity.

### Example 2. Obtaining compounds of FORMULA I with leishmanicidal activity

In another embodiment of the invention, the compounds of formula (I) have been prepared by means of the following method B as described in Figure 1:
Obtaining the compounds of formula (I) SG-010, wherein R1 and R3 are H and R2 is 2-methylbutane, started with a solution of 2,5-dimethoxytoluene (50.0 mg, 0.3 mmol) in 10 ml of ether at a temperature of 0°C, and a solution of 0.5 ml of 1.7 M n-butyllithium (0.5 mmol) in hexane was added. After 10 minutes at 0°C, the reaction was kept under stirring for 24 hours at room temperature. Subsequently, the mixture was cooled to 0°C again, and 2-chloro-2-methylbutane was added dropwise. After 30 minutes of stirring, the reaction was stopped with a saturated ammonium chloride solution, diluted with water and extracted with ethyl acetate (Sanchez *et al.* 1985).

Next, liquid-liquid extraction was performed with ethyl ether and it was dried with MgSO₄. The solvent was evaporated under reduced pressure and the product was fractionated by means of silica gel column chromatography using *n*-hex:AcOEt (99:1 - 97:3) as an eluent. The fractions obtained were concentrated, and the fraction that showed signs of 2,5-dimethoxytoluene ring substitution was identified according to ¹H NMR spectra.

As in Example 1, the last reaction step consists of deprotection of the methoxyl groups and formation of quinones by adding silver oxide dissolved in nitric acid. The resulting products (compounds of formula I) were purified by means of silica column chromatography, followed by purification via HPLC (liquid chromatography) using *n*-hex:AcOEt (99:1 - 0:100) as an eluent. Identification was performed by means of nuclear magnetic resonance spectroscopic techniques and confirmed by mass spectrometry.

The compound SG-010 was obtained as a translucent crystal; HRESIMS m/z 191.1072 [M-H]-(calc. C₁₂H₁₅O₂, 191.1072) ¹H NMR (600 MHz, CDCl₃) δ 0.87 (3H, m, H-11), 1.20 (3H, s, H-8), 1.20 (3H, s, H-12), 1.70 (2H, dd, J = 5.0, 3.6 Hz, H-10), 2.01 (3H, d, J = 1.6 Hz, H-7), 6.52 (1H, s, H-6); ¹³C NMR δ 15.2 (C-7), 27.8 (C-8), 27.8 (C-12), 30.2 (C-11), 35.7 (C-10), 38.5 (C-9), 133.3 (C-6), 135.5 (C-3), 144.3 (C-2), 155.2 (C-5), 187.9 (C-4), 188.8 (C-1).

### Example 3. Evaluation of leishmanicidal activity

The leishmanicidal activity was tested for the compounds of formula I of the first and second aspects or the compounds obtained from the third or fourth aspect, as shown in Table I. For analysing said activity, the compounds synthesised by the method of Example 1 were incubated for 72 hours in the presence of *Leishmania amazonensis* promastigotes and their effect was evaluated with respect to the fluorescence emitted by alamarBlue, such that the less compound is required to eliminate the parasite, the lower the IC50 value is, in other words, the concentration of compound that inhibits 50% of the parasites.

**Table I. Effect of synthetic compounds on murine macrophages and promastigotes of Leishmania amazonensis**

| Compound | Macrophage Toxicity CC₅₀ (µg/ml) | *L. amazonensis* IC₅₀ (µg/ml) | SI (CC₅₀/IC₅₀) |
|---|---|---|---|
| SG-003 | 4.53 ± 0.90 | 0.34 ± 0.01 | 13.30 |
| SG-005 | 0.72 ± 0.01 | 0.13 ± 0.03 | 5.50 |
| SG-007 | 0.52 ± 0.09 | 0.07 ± 0.02 | 7.40 |
| SG-008 | 2.55 ± 0.20 | 0.06 ± 0.01 | 42.50 |
| SG-010 | 4.51 ± 0.47 | 0.12 ± 0.02 | 37.50 |
| SG-011 | 12.27 ± 0.77 | 0.89 ± 0.06 | 13.80 |
| SG-012 | 0.78 ± 0.02 | 0.08 ± 0.01 | 9.75 |
| SG-013 | 0.92 ± 0.05 | 0.09 ± 0.00 | 7.10 |
| SG-014 | 8.95 ± 1.75 | 0.70 ± 0.02 | 24.20 |
| Miltefosine² | 29.42 ± 1.25 | 2.64 ± 0.10 | 11.14 |

| | | | |
|---|---|---|---|
| ¹ Natural quinones;² Reference Drug, NS: non-selective. | | | |

The compounds of formula (I) of the invention exhibit an IC50 between 0.06 and 0.89 µg/ml, all of them exhibiting greater leishmanicidal activity than the reference drug miltefosine.

To assess toxicity, all compounds were evaluated against mouse cells for 24 hours by means of the alamarBlue assay. The compounds had a mean cytotoxic concentration (CC₅₀) between 0.52 and 12.27 µg/ml. The higher the value of CC₅₀, the greater the amount of compound required to damage mouse cells, therefore, the lower the toxicity of the compound.

The ratio of CC₅₀ to IC₅₀ is the selectivity index (SI). It shows the selectivity of the compound on parasites in such a way that an index showing the ratio between the dose required to damage the mouse cell and the dose required to inhibit the parasite can be established. The compounds of formula I have good SI values, most of them being better than the SI value of miltefosine.

## Claims

1. A compound of formula (I), the isomeric forms thereof and the salts of same, 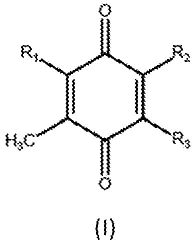
wherein R1 and R3 are hydrogen and R2 is selected from the list consisting of cyclopentyl, cycloheptyl, and 3-methyl-pentyl;
or wherein R1 and R2 are hydrogen and R3 is selected from the list consisting of cyclopentyl, cyclohexyl and cycloheptyl.

2. The compound according to claim 1, wherein the compound is selected from the list of structures consisting of: 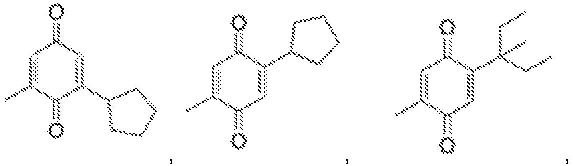 or 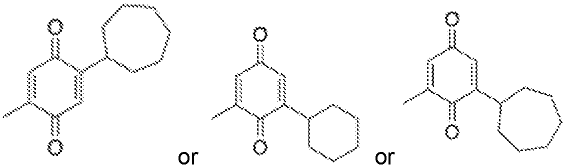 named SG-003:2-cyclopentyl-6-methylcyclohexa-2,5-diene-1,4-dione; SG-005: 2-cyclopentyl-5-methylcyclohexa-2,5-diene-1,4-dione; SG-011: 2-methyl-5-(3-methylpentan-3-yl)cyclohexa-2,5-diene-1,4-dione; or SG-013: 2-cycloheptyl-5-methylcyclohexa-2,5-diene-1,4-dione; or SG-007: 2-cyclohexyl-6-methylcyclohexa-2,5-diene-1,4-dione; or SG-014: 2-cycloheptyl-6-methylcyclohexa-2,5-diene-1,4-dione, respectively.

3. A compound of formula (I), the isomeric forms thereof and the salts of same, for use as a medicinal product, 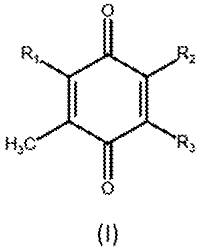
wherein R1 and R3 are hydrogen and R2 is selected from the list consisting of cyclopentyl, cyclohexyl, cycloheptyl, 3-methyl-pentyl, isopentanyl or adamantane;
or wherein R1 and R2 are hydrogen and R3 is selected from the list consisting of cyclopentyl, cyclohexyl and cycloheptyl.

4. The compound according to claim 3, wherein the compound is selected from the list of structures consisting of: 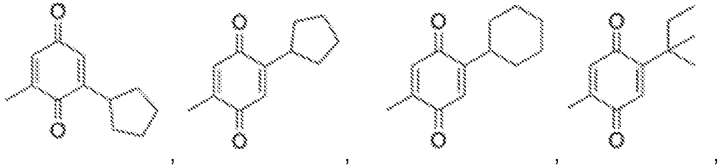 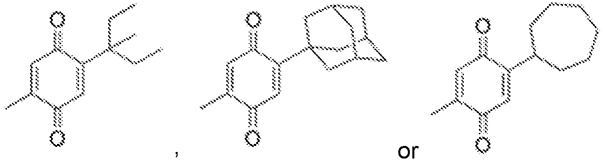 or 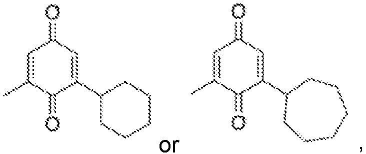 named SG-003: 2-cyclopentyl-6-methylcyclohexa-2,5-diene-1,4-dione; SG-005: 2-cyclopentyl-5-methylcyclohexa-2,5-diene-1,4-dione; SG-008: 2-cyclohexyl-5-methylcyclohexa-2,5-diene-1,4-dione; SG-010: 2-methyl-5-(*tert*-pentyl)cyclohexa-2,5-diene-1,4-dione; SG-011: 2-methyl-5-(3-methylpentan-3-yl)cyclohexa-2,5-diene-1,4-dione; SG-012: 2-((3r,5r,7r)-adamantane-1-yl)-5-methylcyclohexa-2,5-diene-1,4-dione; or SG-013: 2-cycloheptyl-5-methylcyclohexa-2,5-diene-1,4-dione; or SG-007: 2-cyclohexyl-6-methylcyclohexa-2,5-diene-1,4-dione; or SG-014: 2-cycloheptyl-6-methylcyclohexa-2,5-diene-1,4-dione, respectively.

5. The compound of formula (I) according to claim 3 or 4, for use as a human or veterinary medicinal product.

6. The compound according to claim 5 for use in the treatment of leishmaniasis.

7. The compound according to claim 6, wherein leishmaniasis is caused by *Leishmania spp.*

8. A pharmaceutical composition comprising a compound according to any of claims 1-7 and at least one pharmaceutically acceptable excipient.

9. The pharmaceutical composition according to the preceding claim, comprising the compound of formula (I) in a range by weight in the composition of 0.1-5% w/w.

10. The pharmaceutical composition according to claim 8, comprising:
• 0.1-5% by weight of the compound of formula (I),
• 35-45% by weight of polyacrylamide,
• 15-25% by weight of C13-14 isoparaffin, and
• 3-8% surfactant, the surfactant preferably being laureth-7.

11. The pharmaceutical composition according to any of claims 8-10 for topical use, in liquid form, in cream form or in gel form.

12. The pharmaceutical composition according to any of claims 8-11, wherein the composition is a composition for oral use in tablet, capsule, granule, pill or freeze-dried form.

13. A method of obtaining the compounds of formula (I) according to any of claims 1-4, which comprises at least the following steps:
i) providing a mixture of 2,5-dimethoxytoluene in the presence of aluminium trichloride and alkyl chloride in a nitrogenous solvent, preferably nitromethane;
ii) stirring the mixture from the previous step for at least 30 minutes at a temperature range of - 10°C to 10°C, preferably 0°C, under anhydrous conditions and in an inert atmosphere;
iii) adding water to the product of the previous reaction to stop the reaction followed by extraction of the products obtained in the previous step with an organic solvent;
iv) adding silver oxide and nitric acid sequentially to the products obtained in the previous step;
v) optionally adding water to the product of the previous reaction followed by organic solvent extraction;
vi) optionally, performing a purification step.

14. The method of obtaining the compounds of formula (I) according to the preceding claim wherein, the organic solvent of step iii) is selected from a list consisting of hexane, pentane, ethyl acetate or dichloromethane.

15. The method of obtaining the compounds of formula (I) according to any of claims 13-14, wherein the organic solvent of step v) is selected from the list consisting of dichloromethane, hexane, diethyl ether, acetone, methanol, ethanol, isopropanol tetrahydrofuran and diethyl amine.

16. The method of obtaining the compounds of formula (I) according to any of claims 1-4, which comprises at least the following steps:
i) providing a mixture of 2,5-dimethoxytoluene organic solvent at a temperature in the range of -5°C to 10°C, preferably at 0°C;
ii) adding to the previous mixture a solution of n-butyllithium in organic solvent at a temperature in a range of -5°C to 10°C for at least 10 minutes; preferably 0°C for 10 minutes;
iii) increasing the temperature of the previous mixture to a range of 20°C to 30°C;
iv) cooling the product from the previous step to a temperature in the range of -10°C to 5°C, followed by the dropwise addition of alkyl chloride;
v) adding ammonium chloride to the product from the previous step, followed by organic solvent extraction;
vi) adding silver oxide and nitric acid sequentially to the products obtained in the previous step;
vii) optionally adding water to the product of the previous reaction followed by organic solvent extraction;
viii) optionally performing a purification step.

17. The method according to the preceding claim, wherein the alkyl of step iv) is a linear or branched C₁-C₁₀ alkyl.

18. The method according to any of claims 16 to 17, wherein the organic solvent of steps i) and vii) is selected from the list consisting of dichloromethane, hexane, diethyl ether, acetone, methanol, ethanol, isopropanol tetrahydrofuran and diethyl amine.
